# EUROPEAN PATENT APPLICATION

(11) **EP 4 671 236 A1**
(43) Date of publication of application: **31.12.2025**
(21) Application number: 24759848.5
(22) Date of filing: 19.02.2024
(51) Int. Cl.: C07D 209/86, C09K 11/06, H05B 33/22, H10K 50/15, H10K 50/16

(54) **ARYLAMINE COMPOUND, ORGANIC ELECTROLUMINESCENCE ELEMENT, AND ELECTRONIC DEVICE**

(30) Priority: 21.02.2023 KR 20230022722
(71) Applicant: Hodogaya Chemical Co., Ltd., Tokyo, 105-0021 (JP)
(72) Inventor: KASE, Kouki, Tokyo 105-0021 (JP); LIM, Jae-Geon, Tokyo 105-0021 (JP); CHOI, Yeong-Tae, Tokyo 105-0021 (JP); SHIN, Heung-Seob, Tokyo 105-0021 (JP); IZUMIDA, Junichi, Tokyo 105-0021 (JP); HAYASHI, Shuichi, Tokyo 105-0021 (JP)
(74) Representative: dompatent
(86) International application number: PCT/IB2024/051550
(87) International publication number: WO 2024/176075

(57) **Abstract**

The present disclosure aims to provide, as a material for highly efficient and durable organic EL elements, an organic compound having superior characteristics such as excellent hole injection and transport performances, electron blocking ability, and high stability in a thin-film state. Additionally, the present disclosure aims to provide a highly efficient and durable organic EL element by using the compound.

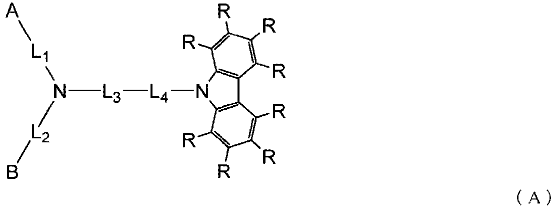

In general formula (A),
A and B each represents a substituted or unsubstituted aromatic hydrocarbon group, a substituted or unsubstituted aromatic heterocyclic group, or a substituted or unsubstituted fused polycyclic aromatic group;
L1 to L4 each represents a single bond, a substituted or unsubstituted divalent aromatic hydrocarbon group, a substituted or unsubstituted divalent aromatic heterocyclic group, or a substituted or unsubstituted divalent fused polycyclic aromatic group; and
Rs may be the same or different, and each of the Rs represents a hydrogen atom, a deuterium atom, a substituted or unsubstituted aromatic hydrocarbon group, a substituted or unsubstituted aromatic heterocyclic group, or a substituted or unsubstituted fused polycyclic aromatic group.

The arylamine compound of the present disclosure has excellent heat resistance and good hole transport ability. Organic EL elements incorporating the compound into their hole transport layer, electron blocking layer, light emitting layer, or hole injection layer exhibit favorable device characteristics.

## Description

### TECHNICAL FIELD

The present disclosure relates to compounds and elements suitable for organic electroluminescence elements (hereinafter abbreviated as "organic EL elements"), which are self-luminous elements suitable for various display applications. Specifically, the present disclosure pertains to arylamine compounds and organic EL elements employing such compounds.

### BACKGROUND ART

Organic EL elements, being self-luminous, are brighter and exhibit superior visibility compared to liquid crystal devices, and can provide clearer displays. Thus, extensive research has been conducted in this field.

In 1987, C.W. Tang et al. of Eastman Kodak developed a device having a layered structure in which various functions were assigned to different materials, making organic EL elements practically applicable. By layering an electron-transporting phosphor and a hole-transporting organic material, and injecting both charges into the phosphor layer to induce light emission, they obtained a high luminescence exceeding 1000 cd/m² at a voltage below 10V *(see* Patent Literatures 1 and 2, for example).

Since then, significant improvements have been made to commercialize organic EL elements. Various roles of a layered structure were further subdivided to produce electroluminescence elements in which an anode, a hole injection layer, a hole transport layer, a light emitting layer, an electron transport layer, an electron injection layer, and a cathode are sequentially provided in that order on a substrate, which enabled high efficiency and high durability to be achieved (*see* Non-Patent Literature 1, for example).

Additionally, to further enhance luminous efficiency, attempts have been made to utilize triplet excitons, and the use of phosphorescent compounds has been explored (*see* Non-Patent Literature 2, for example). Furthermore, devices utilizing light emission by thermally activated delayed fluorescence (TADF) are being developed. In 2011, Adachi et al. from Kyushu University achieved an external quantum efficiency of 5.3% with a device utilizing a TADF material (*see* Non-Patent Literature 3, for example).

The light emitting layer can be fabricated by doping a charge-transporting compound, commonly referred to as a host material, with a fluorescent compound, a phosphorescent compound, or a material exhibiting delayed fluorescence. As described in the aforementioned non-patent literature, the choice of organic materials in an organic EL element significantly impacts the properties of the EL element, such as its efficiency or durability (*see* Non-Patent Literatures 1 to 3, for example).

In organic EL elements, light emission occurs as charges injected from both electrodes recombine in the light emitting layer. It is critical to achieve efficient delivery of holes and electrons to the light emitting layer, and therefore, it is necessary to design such elements to have excellent carrier balance. By using materials having characteristics that improve hole injecting ability for supplying holes injected from the anode to the light emitting layer and improve electron blocking ability for blocking electrons injected from the cathode, the probability of holes and electrons recombining within the light emitting layer is improved. Furthermore, high luminous efficiency can be achieved by confining excitons generated in the light emitting layer. To this end, the role of hole transport materials is crucial. Thus, there is a need for hole transport materials having good hole injection performance, high hole mobility, high electron blocking ability, and high durability against electrons.

Additionally, heat resistance and amorphousness of materials are also important for the device lifespan of such elements. Materials with low heat resistance undergo thermal decomposition even at low temperatures by the heat generated during device operation, leading to deterioration of the materials. In the case of materials with low amorphousness, crystallization in thin films takes place even in a short period of time, leading to deterioration of EL elements. Therefore, materials used in such elements are required to exhibit high heat resistance and good amorphousness.

Hole-transport materials that have been used in organic EL elements include N,N'-diphenyl-N,N'-di(α-naphthyl)benzidine (NPD) and various aromatic amine derivatives (*see* Patent Literatures 1 and 2, for example). While NPD demonstrates good hole transport ability, its glass transition temperature (Tg), which is an indicator of heat resistance, is as low as 96°C, resulting in deterioration of device characteristics due to crystallization under high-temperature conditions (*see* Non-Patent Literature 4, for example).

Moreover, among the aromatic amine derivatives described in the aforementioned patent literature, some compounds exhibit excellent hole mobility of 10⁻³ cm²/Vs or higher (*see* Patent Literatures 1 and 2, for example). However, their electron blocking capabilities are insufficient, allowing some electrons to escape from the light emitting layer, thereby limiting the potential for improving luminous efficiency. To achieve further improvements in device efficiency, materials with higher electron blocking ability, enhanced thin-film stability, and higher heat resistance are required. Further, although highly durable aromatic amine derivatives have been reported (*see* Patent Literature 3, for example), they were used as charge transport materials in electrophotographic photoreceptors, not in organic EL elements.

To address these challenges, substituted carbazole structures and arylamine compounds have been proposed as compounds with improved characteristics such as heat resistance and hole injection performance (*see* Patent Literatures 4 and 5, for example). While EL elements using these compounds in the hole injection layer or hole transport layer have shown improvements in device lifespan and luminous efficiency, the improvements are insufficient. There is still a demand for further advances in lowering the driving voltage, increasing the luminous efficiency, and extending the device lifespan.

### [Prior Art Documents]

### [Patent Literature]

(Patent Literature 1) US Patent No. 5,792,557
(Patent Literature 2) US Patent No. 5,639,914
(Patent Literature 3) US Patent No. 7,759,030
(Patent Literature 4) JP 2009-076817A1
(Patent Literature 5) JP Patent No. 6674892
(Patent Literature 6) EP Patent No. 2,684,932
(Patent Literature 7) KR Patent No. 102452568
(Patent Literature 8) KR Patent No. 102242490

### [Non-Patent Literature]

(Non-Patent Literature 1) The Japan Society of Applied Physics, 9th Lecture Preprints, pp. 55-61 (2001)
(Non-Patent Literature 2) The Japan Society of Applied Physics, 9th Lecture Preprints, pp. 23-31 (2001)
(Non-Patent Literature 3) Appl. Phys. Let., 98, 083302 (2011)
(Non-Patent Literature 4) Organic EL Symposium, 3rd Regular Meeting Preprints, pp. 13-14 (2006)

### DISCLOSURE OF INVENTION

### TECHNICAL PROBLEM

The present disclosure aims to provide, as a material for highly efficient and durable organic EL elements, a material for organic EL elements that has (1) excellent hole injection and transport performances, (2) electron blocking ability, (3) high stability in a thin-film state, and (4) excellent durability.

The present disclosure also aims to provide, by using the material of the present disclosure, organic EL elements having (1) high luminous efficiency and power efficiency, (2) low turn-on voltage and practical driving voltage, and (3) extended lifespan.

### TECHNICAL SOLUTION

To achieve the above-described goals, the inventors of the present disclosure took note of the fact that arylamine compounds have excellent hole injection/transport ability, thin-film stability, and durability, and by pursuing optimization of the substitution positions and substituents on a carbazolyl group, they were able to greatly improve characteristics of the material. With organic EL elements also, the inventors have succeeded in improving luminous efficiency and power efficiency, which enables the turn-on voltage and practical driving voltage to be suppressed and a lifespan surpassing those of conventional devices to be realized in turn. In this way, the present disclosure has been accomplished.

Specifically, according to the present disclosure, the following arylamine compound and organic EL elements and electronic devices using the compound are provided.
1) An arylamine compound represented by general formula (A): 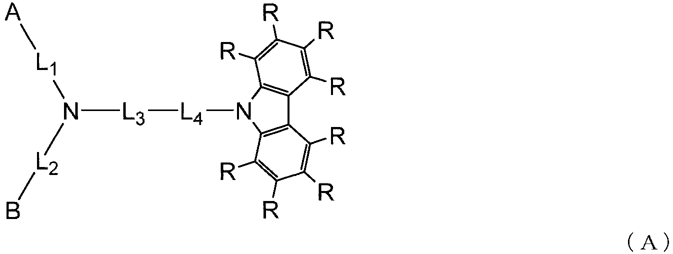 In general formula (A), A and B each represents
   a substituted or unsubstituted aromatic hydrocarbon group,
   a substituted or unsubstituted aromatic heterocyclic group,
   or a substituted or unsubstituted fused polycyclic aromatic group;
   L₁ to L₄ each represents
   a single bond,
   a substituted or unsubstituted divalent aromatic hydrocarbon group,
   a substituted or unsubstituted divalent aromatic heterocyclic group,
   or a substituted or unsubstituted divalent fused polycyclic aromatic group; and
   Rs may be the same or different, and each of the Rs represents
   a hydrogen atom, a deuterium atom,
   a substituted or unsubstituted aromatic hydrocarbon group,
   a substituted or unsubstituted aromatic heterocyclic group,
   or a substituted or unsubstituted fused polycyclic aromatic group,
   provided that at least one of the Rs is a substituted aromatic hydrocarbon group.
2) The arylamine compound as set forth in 1), characterized in that,
   at least one of the Rs in general formula (A) represents
   a deuterated phenyl group.
3) The arylamine compound as set forth in 1) or 2), characterized in that the compound is represented by general formula (B), (C), (D), or (E): 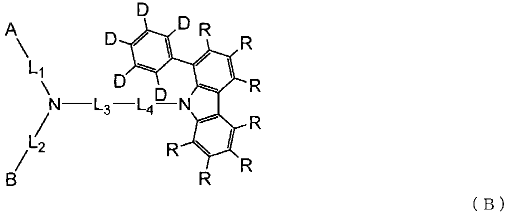 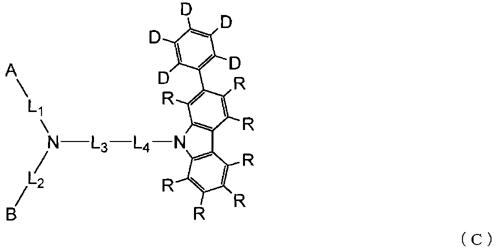 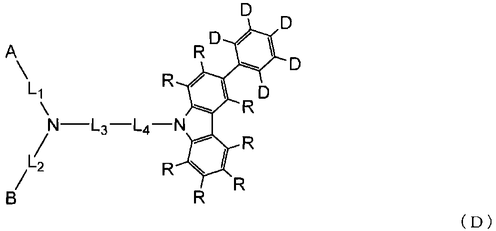 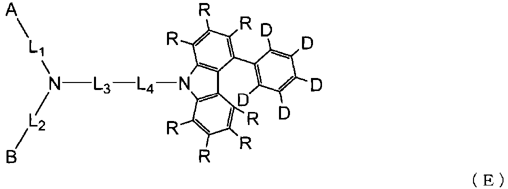 wherein A, B, R, and L₁ to L₄ in general formula (B), (C), (D), or (E) are as defined in general formula (A).
4) The arylamine compound as set forth in 3), characterized in that
   R in general formula (B), (C), (D), or (E) represents
   a hydrogen atom or a deuterium atom.
5) The arylamine compound as set forth in 4), characterized in that
   L₁ to L₄
   in general formula (B), (C), (D), or (E) each represents
   a single bond,
   a substituted or unsubstituted phenylene group,
   a substituted or unsubstituted naphthylene group,
   or a substituted or unsubstituted biphenylene group.
6) The arylamine compound as set forth in 5), characterized in that
   L₄
   in general formula (B), (C), (D), or (E) represents
   a substituted or unsubstituted 1,2-phenylene group,
   a substituted or unsubstituted 1,3-phenylene group,
   a substituted or unsubstituted 1,4-phenylene group,
   a substituted or unsubstituted 1,2-naphthylene group,
   a substituted or unsubstituted 1,3-naphthylene group,
   or a substituted or unsubstituted 1,4-naphthylene group.
7) The arylamine compound as set forth in 6), characterized in that
   L₄
   in general formula (B), (C), (D), or (E) represents
   an unsubstituted 1,2-phenylene group,
   an unsubstituted 1,3-phenylene group,
   an unsubstituted 1,4-phenylene group,
   an unsubstituted 1,2-naphthylene group,
   an unsubstituted 1,3-naphthylene group,
   or a substituted or unsubstituted 1,4-naphthylene group.
8) An organic EL element having a pair of electrodes and at least one organic layer sandwiched therebetween, characterized in that the organic layer comprises the arylamine compound as set forth in 1) or 2).
9) The organic EL element as set forth in 8), characterized in that the organic layer is a hole transport layer.
10) The organic EL element as set forth in 8), characterized in that the organic layer is an electron blocking layer.
11) The organic EL element as set forth in 8), characterized in that the organic layer is a hole injection layer.
12) The organic EL element as set forth in 8), characterized in that the organic layer is a light emitting layer.
13) An electronic device having a pair of electrodes and at least one organic layer sandwiched therebetween, characterized in that the organic layer comprises the arylamine compound as set forth in 1) or 2).

Specific examples
of the "aromatic hydrocarbon group,"
"aromatic heterocyclic group," or
"fused polycyclic aromatic group"
in the "substituted or unsubstituted aromatic hydrocarbon group,"
"substituted or unsubstituted aromatic heterocyclic group," or
"substituted or unsubstituted fused polycyclic aromatic group" represented by A, B, or R in general formula (A) include
a phenyl group, a biphenyl group, a terphenyl group, a naphthyl group, an anthracenyl group, a phenanthrenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a fluorenyl group, a spirobifluorenyl group, an azafluorenyl group, a diazafluorenyl group, an azaspirobifluorenyl group, a diazaspirobifluorenyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group. Other examples include an aryl group having 6 to 30 carbon atoms and a heteroaryl group having 2 to 20 carbon atoms.

Specific examples
of the "substituent"
in the "substituted aromatic hydrocarbon group,"
"substituted aromatic heterocyclic group," or
"substituted fused polycyclic aromatic group"
represented by A, B, or R in general formula (A) include:
   a deuterium atom; a cyano group; a nitro group; a halogen atom such as a fluorine atom, a chlorine atom, a bromine atom, and an iodine atom; a silyl group such as a trimethylsilyl group and a triphenylsilyl group; a linear or branched alkyl group having 1 to 6 carbon atoms, such as a methyl group, an ethyl group, and a propyl group; a linear or branched alkoxy group having 1 to 6 carbon atoms, such as a methoxy group, an ethoxy group, and a propoxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenyl group, a terphenyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and an aromatic heterocyclic group such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group. These substituents may themselves be substituted with any of the substituents listed above. Additionally, any of these substituents and a benzene ring substituted therewith, or a plurality of substituents on the same benzene ring may be linked together via a single bond, a substituted or unsubstituted methylene group, an oxygen atom, or a sulfur atom to form a ring.
   Examples of the "aromatic hydrocarbon group," "aromatic heterocyclic group," or "fused polycyclic aromatic group"
   in the "divalent aromatic hydrocarbon group,"
   "divalent aromatic heterocyclic group,"
   or "divalent fused polycyclic aromatic group"
   in the "substituted or unsubstituted divalent aromatic hydrocarbon group,"
   "substituted or unsubstituted divalent aromatic heterocyclic group,"
   or "substituted or unsubstituted divalent fused polycyclic aromatic group"
   represented by L₁ to L₄ in general formula (A) include
   those listed above as examples of the "aromatic hydrocarbon group," "aromatic heterocyclic group," or "fused polycyclic aromatic group" represented by A, B, or R in general formula (A), and a divalent group may be obtained by removing one hydrogen atom from any of the listed groups.

Examples of the "substituent" in the "substituted divalent aromatic hydrocarbon group," "substituted divalent aromatic heterocyclic group," or "substituted divalent fused polycyclic aromatic group" represented by L₁ to L₄ in general formula (A) include those listed above as examples of the "substituent" in the "substituted aromatic hydrocarbon group," "substituted aromatic heterocyclic group," or "substituted fused polycyclic aromatic group" represented by A, B, or R in general formula (A), and the same holds true for possible forms of those substituents.

In general formula (A), L₄ is preferably an unsubstituted 1,2-phenylene group, an unsubstituted 1,3-phenylene group, or an unsubstituted 1,4-phenylene group, and more preferably an unsubstituted 1,2-phenylene group or an unsubstituted 1,3-phenylene group, and still more preferably an unsubstituted 1,2-phenylene group.

In general formula (A), one or both of A and B are preferably a substituted or unsubstituted phenyl group, a substituted or unsubstituted biphenyl group, a substituted or unsubstituted naphthyl group, a substituted or unsubstituted dibenzofuranyl group, a substituted or unsubstituted dibenzothienyl group, or a substituted or unsubstituted phenanthrenyl group, and more preferably a substituted or unsubstituted phenyl group, a substituted or unsubstituted biphenyl group, or a substituted or unsubstituted naphthyl group, and still more preferably a substituted or unsubstituted phenyl group or a substituted or unsubstituted naphthyl group.

The arylamine compound represented by general formula (A), which is suitable for use in organic EL elements of the present disclosure, is preferably used as a constituent material of a hole injection layer, a hole transport layer, an electron blocking layer, or a light emitting layer in the organic EL elements, and more preferably used as a constituent material of a hole transport layer or an electron blocking layer.

In addition, the arylamine compound of the present disclosure represented by general formula (A) is preferably used, in an electronic device having a pair of electrodes and at least one organic layer sandwiched therebetween, as a constituent material of the organic layer.

### ADVANTAGEOUS EFFECTS

The arylamine compound of the present disclosure represented by general formula (A) has the following advantages over conventional hole transport materials: (1) better hole injection characteristics, (2) greater hole mobility, (3) superior electron-blocking ability, (4) higher electron resistance, (5) more stable thin-film state, and (6) superior heat resistance. By using an arylamine compound of the present disclosure represented by general formula (A) for an organic EL element, it is possible to obtain the following characteristics: (1) high luminous efficiency, (2) low turn-on voltage, (3) low practical driving voltage, and (4) extended lifespan.

The arylamine compound of the present disclosure represented by general formula (A) is excellent in hole injection and transport performances, thin film stability, and durability. As a result, an organic EL element having a hole injection layer and/or a hole transport layer fabricated using the compound as a hole injection material and/or a hole transport material exhibits an improved hole transport efficiency in the light emitting layer, leading to an enhanced luminous efficiency, and a lower driving voltage, improving the durability of the element. This makes it possible to obtain characteristics such as a high efficiency, a low driving voltage, and an extended lifespan.

The arylamine compound of the present disclosure represented by general formula (A) has the characteristics of having an excellent electron blocking ability, having a high electron resistance, and being stable even in thin-film states, thus confining excitons generated in a light emitting layer. As a result, an organic EL element having an electron blocking layer fabricated using the compound as an electron blocking material exhibits an increased probability of hole-electron recombination, which suppresses thermal deactivation, and therefore, has a high luminous efficiency. The organic EL element also has a lower driving voltage and therefore improved current resistance, which leads to an increased maximum luminance.

The arylamine compound of the present disclosure represented by general formula (A) has an excellent hole transport ability and a wide band gap. As a result, an organic EL element having a light emitting layer fabricated using the compound as a host material can exhibit a reduced driving voltage and an improved luminous efficiency when the light emitting layer is formed by incorporating a so-called dopant such as a fluorescence emitter, a phosphorescence emitter, or a delayed fluorescence emitter.

Thus, the arylamine compound of the present disclosure represented by general formula (A) is useful as a constituent material for a hole injection layer, a hole transport layer, an electron blocking layer, or a light emitting layer of an organic EL element and can improve the luminous efficiency, driving voltage and durability of conventional organic EL elements.

In addition, the arylamine compound of the present disclosure represented by general formula (A) can be used not only in organic EL elements but also in other electronic apparatus fields including electrophotographic photoreceptors, image sensors, photoelectric conversion elements, and solar cells.

### BRIEF DESCRIPTION OF DRAWINGS

FIG. 1 shows the structures of compounds (1) to (15), which are arylamine compounds represented by general formula (A).
FIG. 2 shows the structures of compounds (16) to (27), which are arylamine compounds represented by general formula (A).
FIG. 3 shows the structures of compounds (28) to (39), which are arylamine compounds represented by general formula (A).
FIG. 4 shows the structures of compounds (40) to (51), which are arylamine compounds represented by general formula (A).
FIG. 5 shows the structures of compounds (52) to (63), which are arylamine compounds represented by general formula (A).
FIG. 6 shows the structures of compounds (64) to (78), which are arylamine compounds represented by general formula (A).
FIG. 7 shows the structures of compounds (79) to (90), which are arylamine compounds represented by general formula (A).
FIG. 8 shows the structures of compounds (91) to (105), which are arylamine compounds represented by general formula (A).
FIG. 9 shows the structures of compounds (106) to (118), which are arylamine compounds represented by general formula (A).
FIG. 10 shows the structures of compounds (119) to (130), which are arylamine compounds represented by general formula (A).
FIG. 11 shows the structures of compounds (131) to (142), which are arylamine compounds represented by general formula (A).
FIG. 12 shows the structures of compounds (143) to (157), which are arylamine compounds represented by general formula (A).

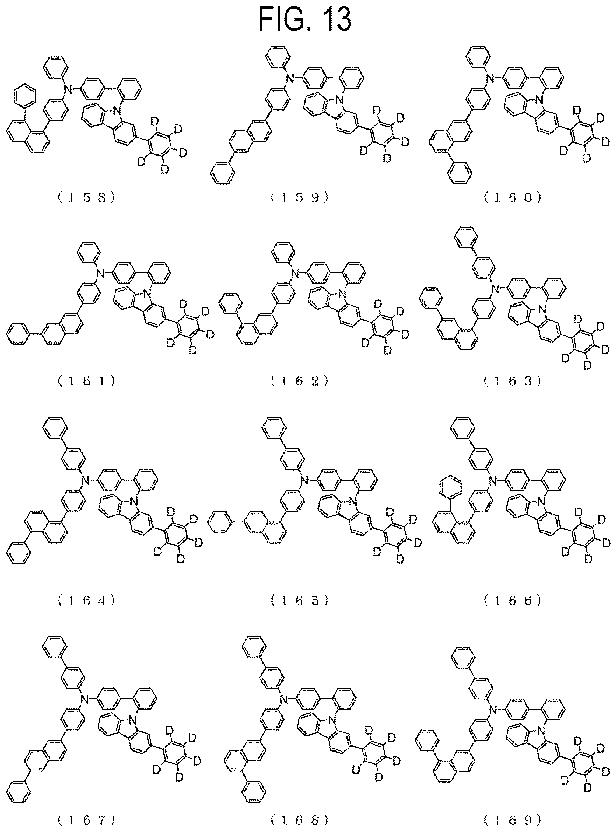

FIG. 13 shows the structures of compounds (158) to (169), which are arylamine compounds represented by general formula (A).
FIG. 14 is a schematic diagram showing the structure of the organic EL elements described in Examples 9 to 14 and Comparative Examples 1 and 2.

### MODES FOR CARRYING OUT THE INVENTION

The arylamine compound of the present disclosure represented by general formula (A), though novel, can be synthesized following well-established methods.

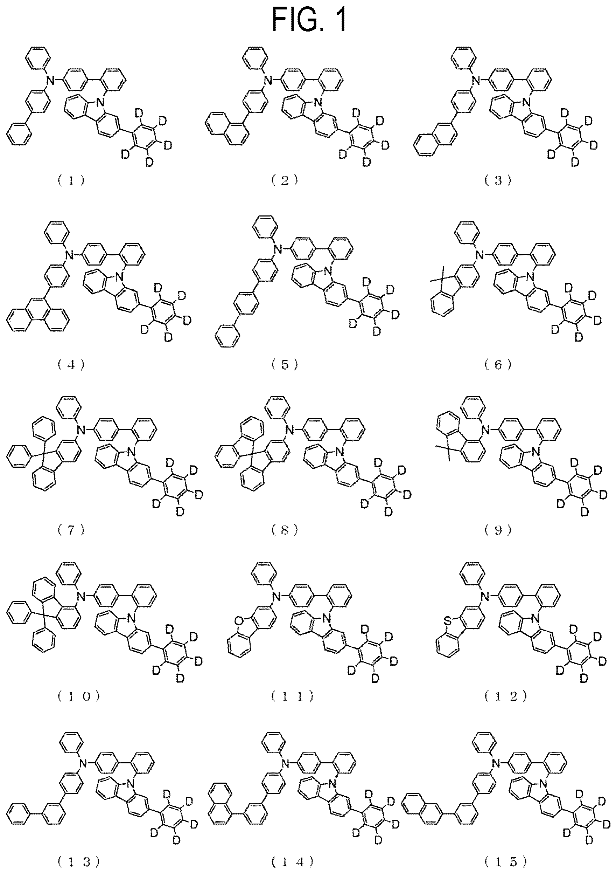

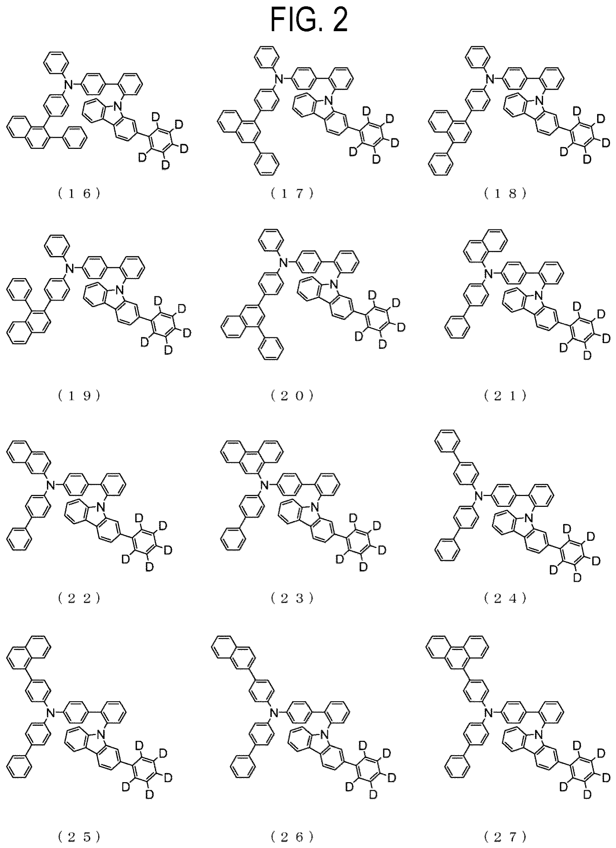

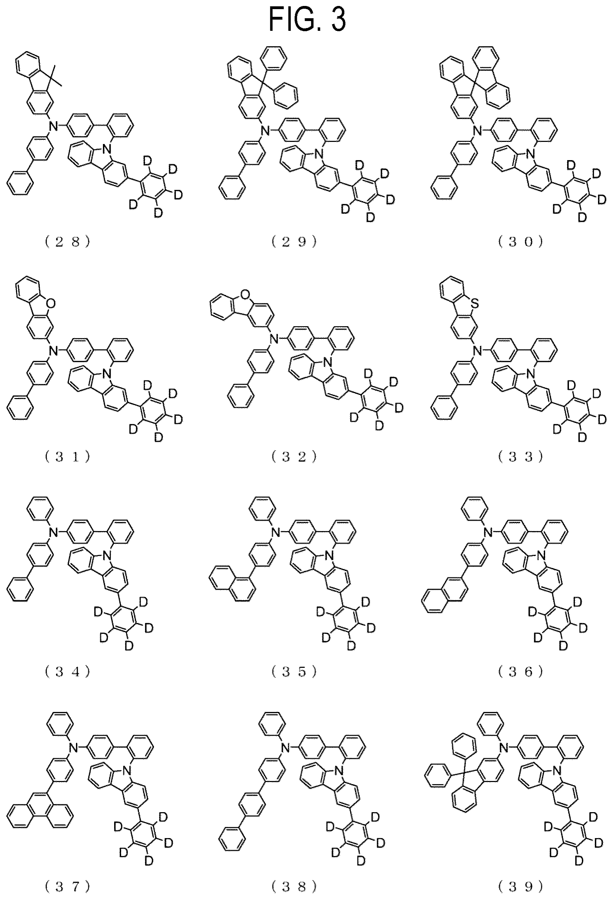

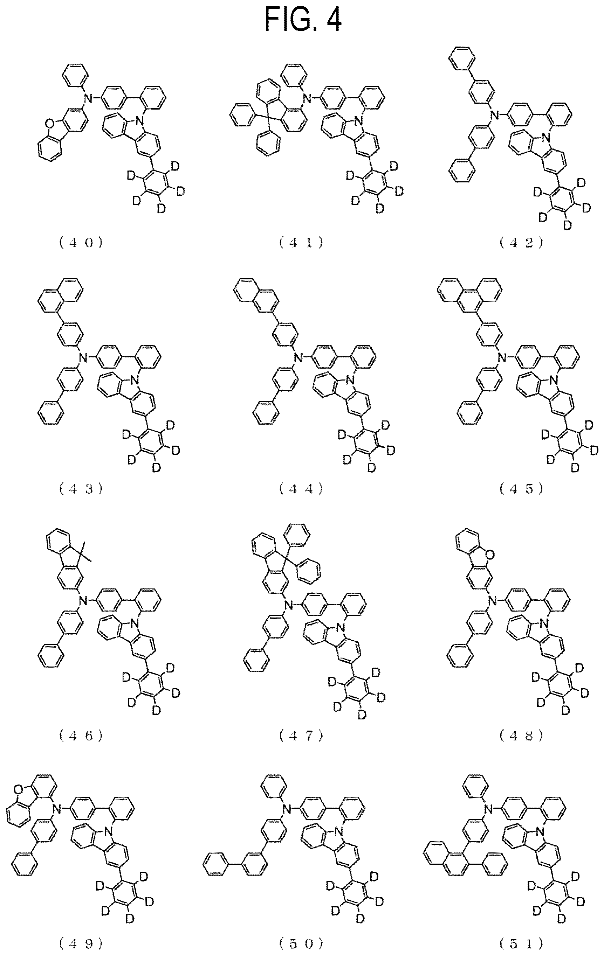

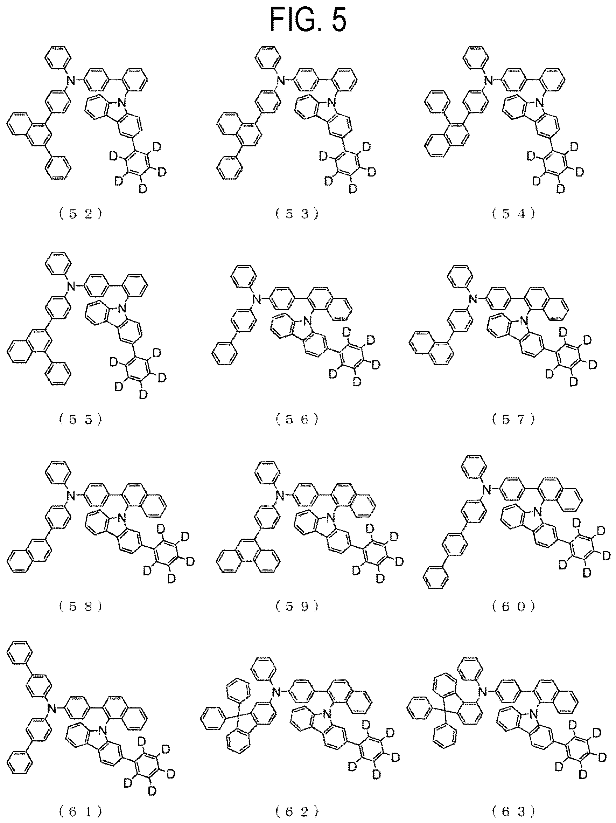

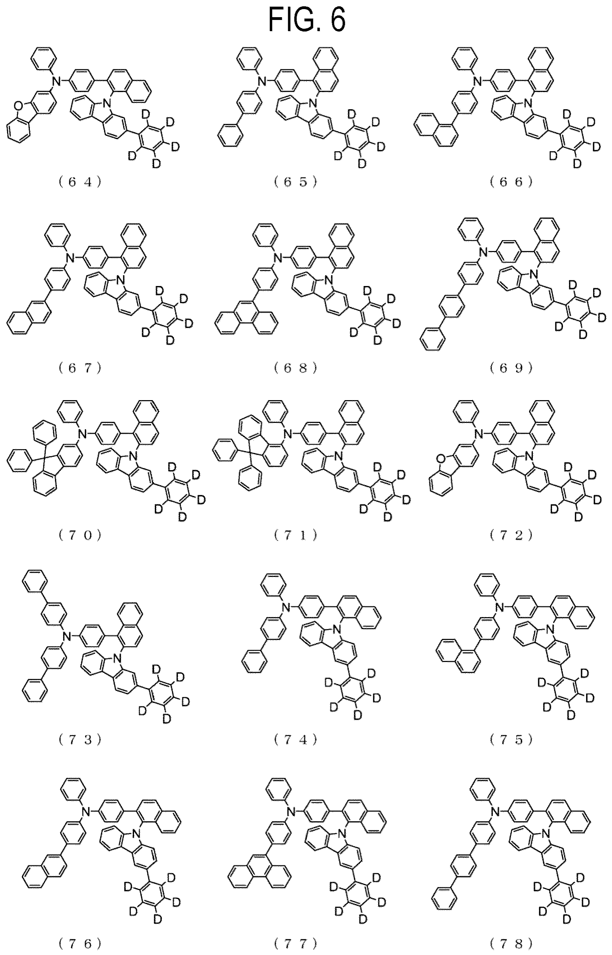

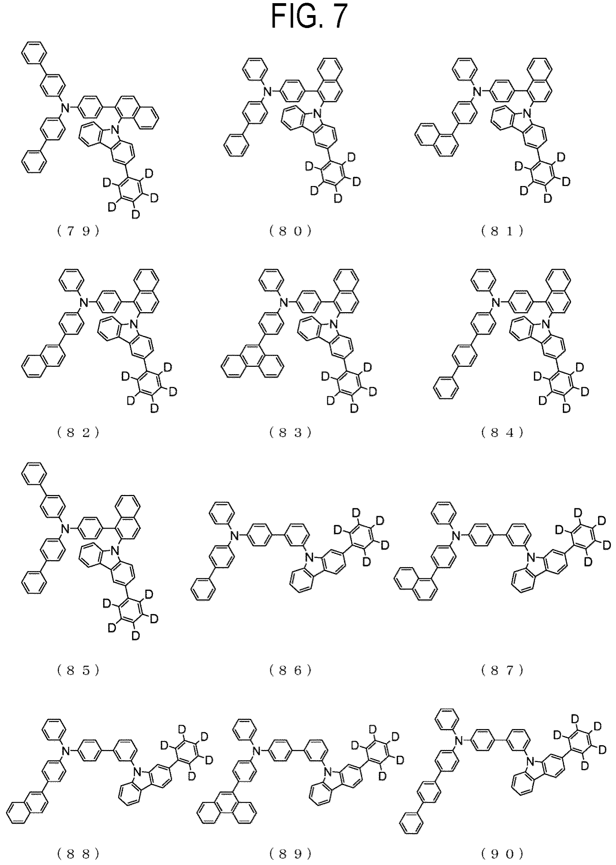

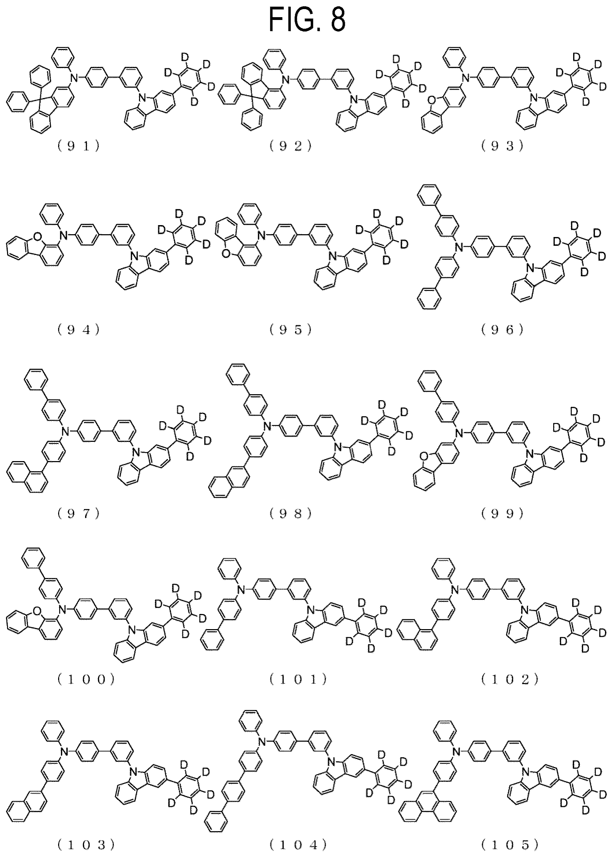

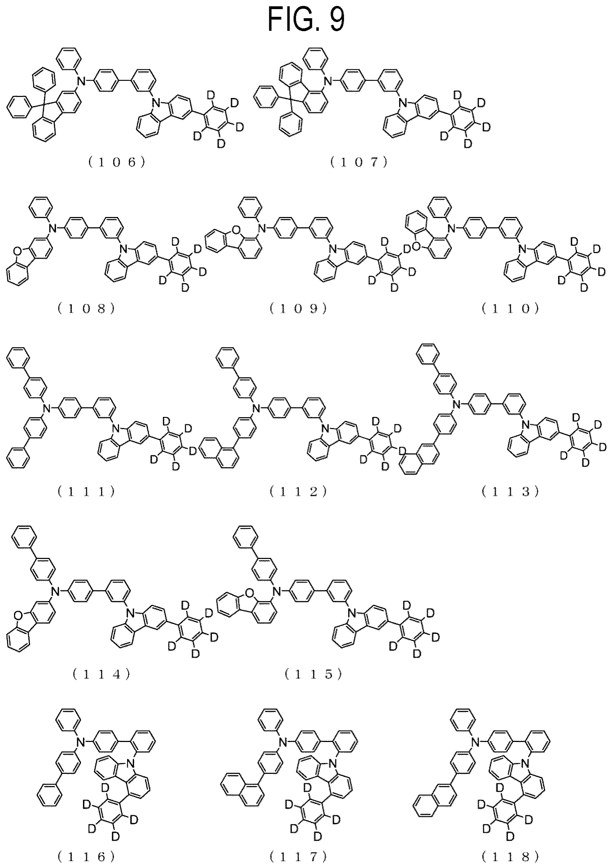

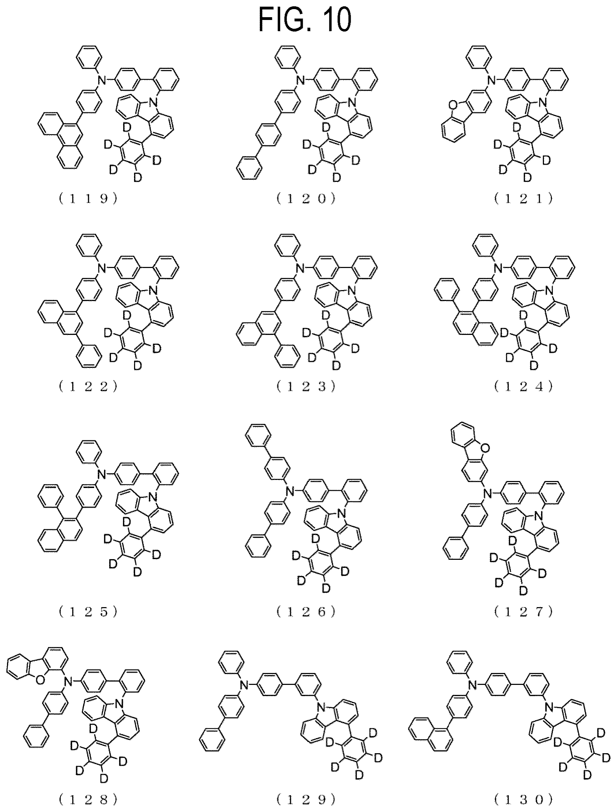

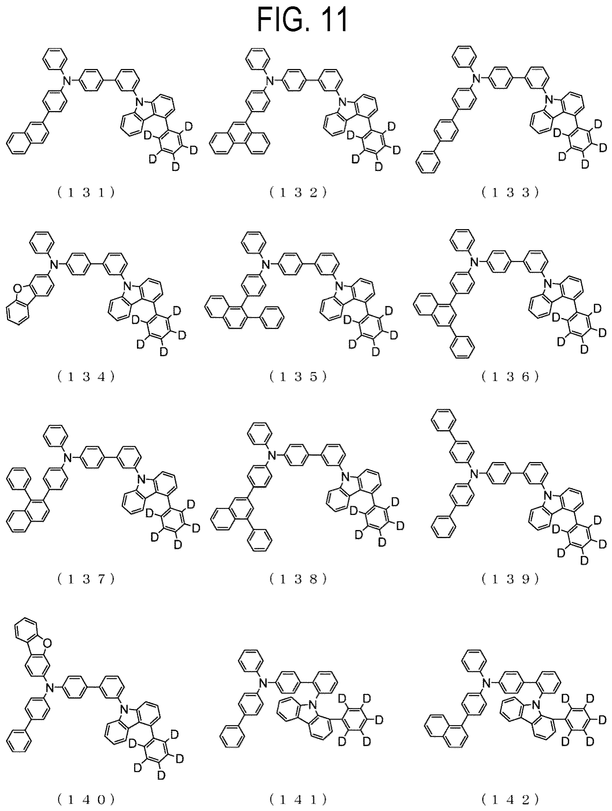

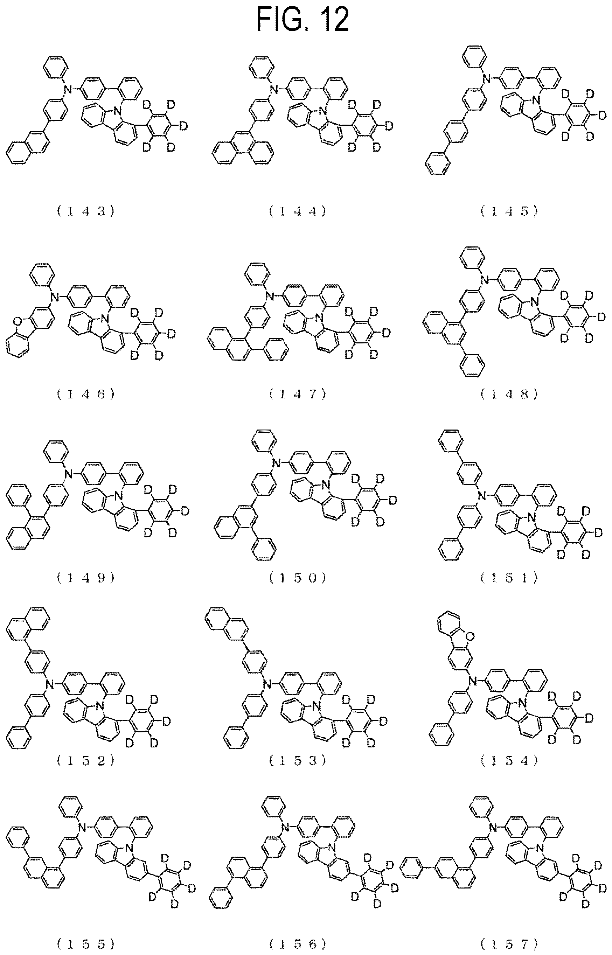

Preferred examples of the arylamine compound represented by general formula (A) suitable for use in organic EL elements of the present disclosure are depicted in FIGS. 1 to 12 but are not limited thereto.

Purification of the arylamine compound of the present disclosure represented by general formula (A) can be conducted using conventional techniques, such as column chromatography graph, adsorption with silica gel, activated carbon, or activated clay, recrystallization or precipitation with a solvent, sublimation, etc., and the final purification was conducted by using sublimation. Compounds were identified by NMR analysis. As relevant physical properties, melting points, glass transition temperatures (Tg), and work functions were determined. The melting point is an index of vapor deposition characteristics, and the glass transition temperature (Tg) reflects thin-film stability. The work function is an index of hole injection, hole transport, or electron blocking ability.

In addition, compounds used for an organic EL element of the present disclosure were purified by column chromatography graph, adsorption with silica gel, activated carbon, or activated clay, recrystallization or precipitation with a solvent, or the like, and then finally purified using sublimation prior to use.

The melting point and the glass transition temperature (Tg) can be measured, for example, by a high-sensitivity differential scanning calorimeter (e.g., DSC3100SA from Bruker AXS) using powdered samples.

The work function can be determined, for example, by creating a 100 nm thin film on an ITO substrate and using an ionization potential measurement device (e.g., PYS-202 from Sumitomo Heavy Industries Ltd.).

The structure of an organic EL element of the present disclosure may be configured to include an anode, a hole injection layer, a hole transport layer, a light emitting layer, an electron transport layer, an electron injection layer, and a cathode sequentially provided on a substrate, optionally with an electron blocking layer provided between the hole transport layer and the light emitting layer or a hole blocking layer provided between the light emitting layer and the electron transport layer. In such multi-layer configurations, a single organic layer may perform the functions of several layers. For example, a single organic layer may be configured to serve both as a hole injection layer and a hole transport layer or as an electron injection layer and an electron transport layer. It is also possible to stack two or more layers having the same function, as in dual-stacked hole transport layers, light emitting layers, or electron transport layers.

The anode material for an organic EL element of the present disclosure includes electrode materials with a high work function, such as ITO or gold.

As the material for a hole injection layer in an organic EL element of the present disclosure, it is preferable to use an arylamine compound having only one triphenylamine moiety in the molecule, such as the arylamine compound of the present disclosure represented by general formula (A). In addition, porphyrin compounds exemplified by copper phthalocyanine, starburst-type triphenylamine derivatives, arylamine compounds having two or more triphenylamine moieties or carbazolyl moieties in the molecule, with the moieties linked together via a single bond or a divalent group containing no heteroatoms, acceptor-type heterocyclic compounds such as hexacyanoazatriphenylene, and coating-type polymeric materials may be used. These materials can be formed into a thin film using a known method such as vapor deposition, spin coating, or inkjet printing.

As the material for a hole injection layer and a hole transport layer in an organic EL element of the present disclosure, it is preferable to use an arylamine compound having only one triphenylamine moiety in the molecule, such as the arylamine compound of the present disclosure represented by general formula (A). In addition, benzidine derivatives such as N,N'-diphenyl-N,N'-di(m-tolyl)-benzidine (TPD), N,N'-diphenyl-N,N'-di(α-naphthyl)-benzidine (NPD), and N,N,N',N'-tetrabiphenylylbenzidine, 1,1-bis[(di-4-tolylamino)phenyl]cyclohexane (TAPC), and arylamine compounds having two or more triphenylamine moieties or carbazolyl moieties in the molecule, with the moieties linked together via a single bond or a divalent group containing no heteroatoms, may be used. These materials may be used individually for film formation, but a mixture of multiple materials may also be used. In each case, the resulting film may be used in the form of a single layer. Furthermore, these materials may also be formed into a layered structure composed of single-material layers, a layered structure composed of mixed-material layers, or a layered structure composed of a single-material layer and a mixed-material layer. Additionally, coating-type polymeric materials, such as poly(3,4-ethylenedioxythiophene)/poly(styrene sulfonate) (PEDOT/PSS), may be used as the material for a hole injection/transport layer. These materials can be formed into a thin film using a known method such as vapor deposition, spin coating, or inkjet printing.

For a hole injection layer or a hole transport layer, it is preferable to use a material obtained by p-doping a material typically used for such layers with trisbromophenylamine hexachloroantimony, a radialene derivative (e.g., as described in Patent Literature 6), or the like. In addition, polymeric compounds containing the structure of a benzidine derivative such as TPD as a partial structure thereof may be employed.

As the material for an electron blocking layer in an organic EL element of the present disclosure, it is preferable to use an arylamine compound of the present disclosure represented by general formula (A). In addition, compounds having an electron blocking effect may be used, for example, carbazole derivatives such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz); and compounds having a triphenylsilyl group and a triarylamine structures, exemplified by 9-[4-(carbazol-9-yl)phenyl]-9-[4-(triphenylsilyl)phenyl]-9H-fluorene. These materials may also serve as the material for a hole transport layer. These materials may be used individually for film formation, but a mixture of multiple materials may also be used. In each case, the resulting film may be used in the form of a single layer. Furthermore, these materials may also be formed into a layered structure composed of single-material layers, a layered structure composed of mixed-material layers, or a layered structure composed of a single-material layer and a mixed-material layer. These materials can be formed into a thin film using a known method such as vapor deposition, spin coating, or inkjet printing.

As the material for a light emitting layer in an organic EL element of the present disclosure, it is preferable to use an arylamine compound of the present disclosure represented by general formula (A). In addition, metal complexes of quinolinol derivatives such as tris(8-quinolinolato)aluminum (Alq₃), various metal complexes, anthracene derivatives, bisstyrylbenzene derivatives, pyrene derivatives, oxazole derivatives, and polyparaphenylenevinylene derivatives may be used. The light emitting layer may be composed of a host material and a dopant material. As the host material, an arylamine compound of the present disclosure represented by general formula (A) or an anthracene derivative is preferably used. In addition to the above-mentioned materials, heterocyclic compounds having an indole ring as part of their fused ring structure, heterocyclic compounds having a carbazole ring as part of their fused ring structure, carbazole derivatives, thiazole derivatives, benzimidazole derivatives, and polydialkylfluorene derivatives may be used. As the dopant material, a heterocyclic compound containing S, B, N, or the like as a ring-constituting element is preferably used. In addition, quinacridone, coumarin, rubrene, perylene, and derivatives thereof, benzopyran derivatives, rhodamine derivatives, and aminostyryl derivatives may be employed. These materials may be used individually for film formation, but a mixture of multiple materials may also be used. In each case, the resulting film may be used in the form of a single layer. Furthermore, these materials may also be formed into a layered structure composed of single-material layers, a layered structure composed of mixed-material layers, or a layered structure composed of a single-material layer and a mixed-material layer. These materials can be formed into a thin film using a known method such as vapor deposition, spin coating, or inkjet printing.

It is also possible to use a phosphorescence emitter as the light-emitting material. Phosphorescence emitters may include metal complexes of iridium or platinum. Examples include green phosphorescence emitters such as Ir(ppy)₃, blue phosphorescence emitters such as FIrpic and FIr6, and red phosphorescence emitters such as Btp₂Ir(acac). As the host material for such cases, a host material having hole injection/transport ability may be used, including arylamine compounds of the present disclosure in addition to carbazole derivatives such as 4,4'-di(N-carbazolyl)biphenyl (CBP), TCTA, and mCP, and a host material having electron transport ability may also be used, including p-bis(triphenylsilyl)benzene (UGH2) and 2,2',2"-(1,3,5-phenylene)-tris(1-phenyl-1H-benzimidazole) (TPBI). High-performance organic EL elements can be fabricated by using such materials.

The doping of a phosphorescent light-emitting material into a host material is preferably carried out by co-deposition, to a level within the range of 1 to 30 percent by weight based on the entire light emitting layer in order to avoid concentration quenching.

Materials showing delayed fluorescence, for example, PIC-TRZ, CC2TA, PXZ-TRZ, and CDCB derivatives such as 4CzIPN, may also be used as the light-emitting material (*see,* for example, Non-Patent Literature 3). These materials can be formed into a thin film using a known method such as vapor deposition, spin coating, or inkjet printing.

As the material for a hole blocking layer in an organic EL element of the present disclosure, compounds having a hole blocking effect can be used, including phenanthroline derivatives such as bathocuproine (BCP), metal complexes of quinolinol derivatives such as bis(2-methyl-8-quinolinolate)-4-(phenylphenolato)aluminum (BAlq), various rare-earth complexes, oxazole derivatives, triazole derivatives, and triazine derivatives. These materials may also serve as the material for an electron transport layer. These materials may be used individually for film formation, but a mixture of multiple materials may also be used. In each case, the resulting film may be used in the form of a single layer. Furthermore, these materials may also be formed into a layered structure composed of single-material layers, a layered structure composed of mixed-material layers, or a layered structure composed of a single-material layer and a mixed-material layer. These materials can be formed into a thin film using a known method such as vapor deposition, spin coating, or inkjet printing.

As the material for an electron transport layer in an organic EL element of the present disclosure, it is preferable to use a benzimidazole derivative, an anthracene derivative, a pyrimidine derivative, or a triazine derivative. In addition, metal complexes of quinolinol derivatives such as Alq₃ and BAlq, various metal complexes, triazole derivatives, oxadiazole derivatives, pyridine derivatives, thiadiazole derivatives, carbodiimide derivatives, quinoxaline derivatives, pyridoindole derivatives, phenanthroline derivatives, and silole derivatives may be used. These materials may be used individually for film formation, but a mixture of multiple materials may also be used. In each case, the resulting film may be used in the form of a single layer. Furthermore, these materials may also be formed into a layered structure composed of single-material layers, a layered structure composed of mixed-material layers, or a layered structure composed of a single-material layer and a mixed-material layer. These materials can be formed into a thin film using a known method such as vapor deposition, spin coating, or inkjet printing.

As the material for an electron injection layer in an organic EL element of the present disclosure, alkali metal salts such as lithium fluoride and cesium fluoride, alkaline earth metal salts such as magnesium fluoride, metal complexes of quinolinol derivatives such as lithium quinolinolate, metal oxides such as aluminum oxide, and metals such as ytterbium (Yb), samarium (Sm), calcium (Ca), strontium (Sr), and cesium (Cs) may be used. The electron injection layer may be omitted, upon appropriate choices of an electron transport layer and a cathode.

Additionally, for an electron injection layer and an electron transport layer, materials obtained by N-doping a material typically used for such layers with a metal such as cesium may be used.

For a cathode in an organic EL element of the present disclosure, metals with a low work function such as aluminum, as well as alloys with an even lower work function such as magnesium-silver alloys, magnesium-indium alloys, and aluminum-magnesium alloys, may be employed as electrode materials.

### [EXAMPLES]

Embodiments of the present disclosure will be described in greater detail with reference to the following Examples, but the present disclosure, without departing from its spirit, is not limited thereto.

### [EXAMPLE 1]

### <Synthesis of N-(4-naphthalen-2-yl-phenyl)-N-{2'-(2-phenyl-d₅-9H-carbazol-9-yl)-[1,1'-biphenyl-4-yl]}-phenylamine (Compound (3))>

In a nitrogen-purged reaction vessel, 9-(2-bromophenyl)-2-(phenyl-d₅)-9H-carbazole (7.7g), N-(4-naphthalen-2-yl-phenyl)-N-{4-(4,4,5,5-tetramethyl-[1,3,2]dioxaborolan-2-yl)phenyl} -phenylamine (9.0g), tetrakis(triphenylphosphine)palladium(0) (0.4g), and potassium carbonate (5.0g) were added and stirred overnight under reflux in a toluene/ethanol/water mixed solvent. After completion of the reaction, methanol was added to the system to precipitate solids, followed by filtration to give a crude product. The crude product was purified by recrystallization with an acetone solvent to give N-(4-naphthalen-2-yl-phenyl)-N-{2'-(2-phenyl-d₅-9H-carbazol-9-yl)-[1,1'-biphenyl-4-yl]}-phenylamine (Compound (3)) as a white powder (3.8g, yield: 30.3%).

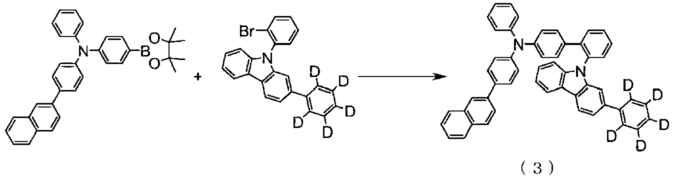

The structure of the resulting white powder was identified by NMR.

The following 31 hydrogen signals were detected by ¹H-NMR(CDCl₃):
δ(ppm) = 8.09(2H), 7.89(2H), 7.86(1H), 7.84(1H), 7.74(2H), 7.68-7.52(4H), 7.47(3H), 7.40(2H), 7.33(1H), 7.26(1H), 7.19(1H), 7.17(1H), 7.10(2H), 6.92(1H), 6.86(2H), 6.81(3H), 6.72(2H).

### [EXAMPLE 2]

### <Synthesis of N,N-bis[1,1'-biphenyl-4-yl]-{2'-(2-phenyl-d₅-9H-carbazol-9-yl)-[1,1'-biphenyl-4-yl]}amine (Compound (24))>

In a nitrogen-purged reaction vessel, N-[1,1'-biphenyl-4-yl]-{2'-(2-phenyl-d₅-9H-carbazol-9-yl)-[1,1'-biphenyl-4-yl]}amine (8.0g), 4-bromo-[1,1'-biphenyl] (3.6g), tris(dibenzylideneacetone)palladium(0) (0.3g), tri-tert-butylphosphine (0.1g), and sodium tert-butoxide (2.7g) were added and stirred overnight under reflux in a xylene solvent. After completion of the reaction, methanol was added to the system to precipitate solids, followed by filtration to give a crude product. The crude product was purified by crystallization with a tetrahydrofuran/acetone mixed solvent to give N,N-bis[1,1'-biphenyl-4-yl]-{2'-(2-phenyl-d₅-9H-carbazol-9-yl)-[1,1'-biphenyl-4-yl]}amine (Compound (24)) as a white powder (5.6g, yield: 55.2%).

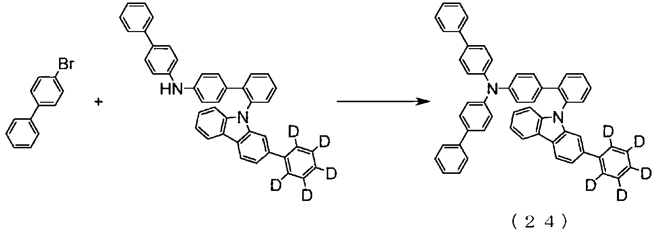

The structure of the resulting white powder was identified by NMR.

The following 33 hydrogen signals were detected by ¹H-NMR(CDCl₃):
δ(ppm) = 8.10(2H), 7.75(1H), 7.65-7.55(4H), 7.50(5H), 7.41(4H), 7.38-7.27(7H), 7.19(2H), 6.88(2H), 6.82(4H), 6.75(2H).

### [EXAMPLE 3]

### <Synthesis of N-[1,1'-biphenyl-4-yl]-N-(4-naphthalen-1-yl-phenyl)-{2'-(2-phenyl-d₅-9H-carbazol-9-yl)-[1,1'-biphenyl-4-yl]}amine (Compound (25))>

In a nitrogen-purged reaction vessel, N-[1,1'-biphenyl-4-yl]-{2'-(2-phenyl-d₅-9H-carbazol-9-yl)-[1,1'-biphenyl-4-yl]}amine (8.0g), 1-(4-bromophenyl)naphthalene (4.4g), tris(dibenzylideneacetone)palladium(0) (0.3g), tri-tert-butylphosphine (0.1g), and sodium tert-butoxide (2.7g) were added and stirred overnight under reflux in a xylene solvent. After completion of the reaction, methanol was added to the system to precipitate solids, followed by filtration to give a crude product. The crude product was purified by crystallization with a tetrahydrofuran/ethyl acetate mixed solvent to give N-[1,1'-biphenyl-4-yl]-N-(4-naphthalen-1-yl-phenyl)-{2'-(2-phenyl-d₅-9H-carbazol-9-yl)-[1,1'-biphenyl-4-yl]}amine (Compound (25)) as a white powder (3.0g, yield: 27.7%).

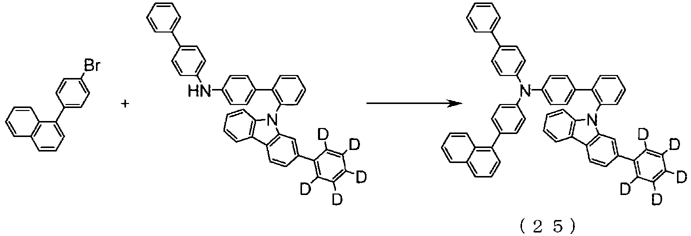

The structure of the resulting white powder was identified by NMR.

The following 35 hydrogen signals were detected by ¹H-NMR(CDCl₃):
δ(ppm) = 8.11(1H), 8.09(1H), 7.95(1H), 7.90(1H), 7.83(1H), 7.77(1H), 7.67-7.54(3H), 7.54-7.26(14H), 7.23(2H), 7.20(2H), 6.89(6H), 6.81(2H).

### [EXAMPLE 4]

### <Synthesis of N-[1,1'-biphenyl-4-yl]-N-(4-naphthalen-2-yl-phenyl)-{2'-(2-phenyl-d₅-9H-carbazol-9-yl)-[1,1'-biphenyl-4-yl]}amine (Compound (26))>

In a nitrogen-purged reaction vessel, N-[1,1'-biphenyl-4-yl]-{2'-(2-phenyl-d₅-9H-carbazol-9-yl)-[1,1'-biphenyl-4-yl]}amine (8.0g), 2-(4-bromophenyl)naphthalene (4.4g), tris(dibenzylideneacetone)palladium(0) (0.3g), tri-tert-butylphosphine (0.1g), and sodium tert-butoxide (2.7g) were added and stirred overnight under reflux in a xylene solvent. After completion of the reaction, methanol was added to the system to precipitate solids, followed by filtration to give a crude product. The crude product was purified by crystallization with a tetrahydrofuran/ethyl acetate mixed solvent to give N-[1,1'-biphenyl-4-yl]-N-(4-naphthalen-2-yl-phenyl)- {2'-(2-phenyl-d₅-9H-carbazol-9-yl)-[1,1'-biphenyl-4-yl] } amine (Compound (26)) as a white powder (6.8g, yield: 62.7%).

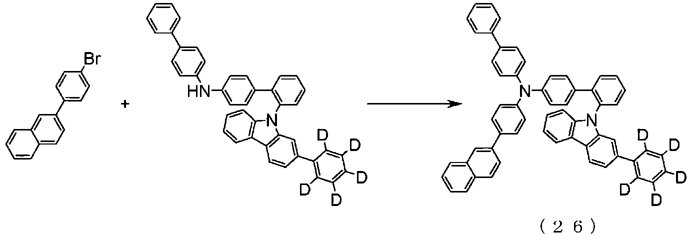

The structure of the resulting white powder was identified by NMR.

The following 35 hydrogen signals were detected by ¹H-NMR(CDCl₃):
δ(ppm) = 8.11(2H), 7.93(1H), 7.88(2H), 7.85(1H), 7.77(1H), 7.68-7.55(4H), 7.50(4H), 7.47-7.26(10H), 7.21(1H), 7.19(1H), 6.87(6H), 6.77(2H).

### [EXAMPLE 5]

### <Synthesis of N-phenyl-N-(4-(8-phenyl-2-naphthalenyl)phenyl)-{2'-(2-phenyl-ds-9H-carbazol-9-yl)-[1,1'-biphenyl-4-yl]}amine (Compound (162))>

In a nitrogen-purged reaction vessel, N-[1,1'-biphenyl-4-yl]-{2'-(2-phenyl-d₅-9H-carbazol-9-yl)-[1,1'-biphenyl-4-yl]}amine (12.0g), 7-(4-chlorophenyl)-1-phenylnaphthalene (8.5g), tris(dibenzylideneacetone)palladium(0) (0.5g), tri-tert-butylphosphine (0.2g), and sodium tert-butoxide (4.7g) were added and stirred overnight under reflux in a xylene solvent. After completion of the reaction, methanol was added to the system to precipitate solids, followed by filtration to give a crude product. The crude product was purified by crystallization with a toluene/ethyl acetate mixed solvent to give N-phenyl-N-(4-(8-phenyl-2-naphthalenyl)phenyl)-{2'-(2-phenyl-d₅-9H-carbazol-9-yl)-[1,1'-biphenyl-4-yl]}amine (Compound (162)) as a white powder (6.2g, yield: 33.0%).

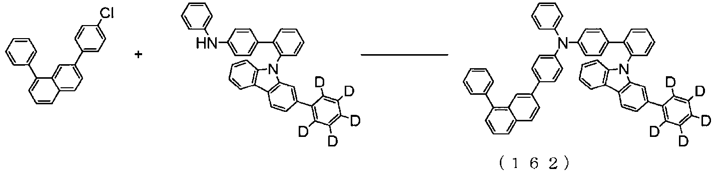

The structure of the resulting white powder was identified by NMR.

The following 35 hydrogen signals were detected by ¹H-NMR(CDCl₃
δ(ppm) = 8.18(2H), 8.08(1H), 7.98(1H), 7.92(1H), 7.83-7.67(5H), 7.64-7.50(7H), 7.47(1H), 7.38(1H), 7.32-7.24(3H), 7.15(3H), 7.09(1H), 6.96(1H), 6.86(2H), 6.69(2H), 6.58(4H).

### [EXAMPLE 6]

### <Synthesis of N-[1,1'-biphenyl-4-yl]-N-(4-(7-phenyl-1-naphthalenyl)phenyl)-{2'-(2-phenyl-d₅-9H-carbazol-9-yl)-[1,1'-biphenyl-4-yl]}amine (Compound (163))>

In a nitrogen-purged reaction vessel, N-[1,1'-biphenyl-4-yl]-{2'-(2-phenyl-d₅-9H-carbazol-9-yl)-[1,1'-biphenyl-4-yl]}amine (9.5g), 1-(4-chlorophenyl)-7-phenylnaphthalene (5.5g), tris(dibenzylideneacetone)palladium(0) (0.3g), tri-tert-butylphosphine (0.1g), and sodium tert-butoxide (3.2g) were added and stirred overnight under reflux in a xylene solvent. After completion of the reaction, the reaction mixture was separated by column chromatography graph (support: silica gel, eluent: dichloromethane/n-heptane) and then purified by crystallization with a tetrahydrofuran/methanol mixed solvent to give N-[1,1'-biphenyl-4-yl]-N-(4-(7-phenyl-1-naphthalenyl)phenyl)-{2'-(2-phenyl-d₅-9H-carbazol-9-yl)-[1,1'-biphenyl-4-yl]}amine (Compound (163)) as a white powder (12.0g, yield: 84.8%).

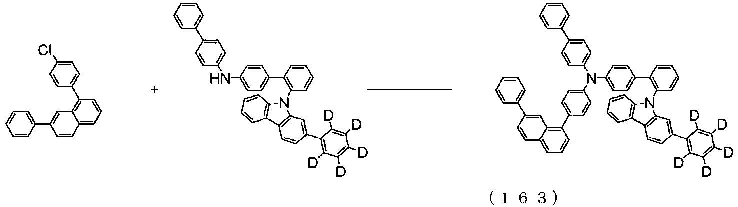

The structure of the resulting white powder was identified by NMR.

The following 39 hydrogen signals were detected by ¹H-NMR(DMSO-d₆):
δ(ppm) = 8.22-8.18(2H), 8.10(1H), 8.06(1H), 7.97(1H), 7.88-7.82(2H), 7.75-7.69(3H), 7.64(2H), 7.60-7.53(4H), 7.47-7.37(9H), 7.32-7.26(4H), 7.17(1H), 7.12(1H), 6.91(2H), 6.79(4H), 6.72(2H).

### [EXAMPLE 7]

The melting point and glass transition temperature of the arylamine compounds represented by general formula (A) were measured using a high-sensitivity differential scanning calorimeter (DSC3100SA from Bruker AXS). The results are summarized in Table 1.

**TABLE 1**

| | Melting Point (°C) | Glass transition temperature (°C) |
|---|---|---|
| Compound (3) | - | 112 |
| Compound (24) | 259 | 121 |
| Compound (25) | - | 128 |
| Compound (26) | - | 126 |
| Compound (162) | - | 127 |
| Compound (163) | - | 134 |

The arylamine compounds represented by general formula (A) have glass transition temperatures above 100°C, indicating that they are stable in a thin-film state.

### [EXAMPLE 8]

A film (thickness: 100 nm) of each of the arylamine compounds represented by general formula (A) was deposited on an ITO substrate, and the work function (in eV) was measured using an ionization potential measurement device (PYS-202 from Sumitomo Heavy Industries, Ltd.). The results are shown in Table 2.

**TABLE 2**

| | Work function (eV) |
|---|---|
| Compound (3) | 5.67 |
| Compound (24) | 5.66 |
| Compound (25) | 5.64 |
| Compound (26) | 5.63 |
| Compound (162) | 5.66 |
| Compound (163) | 5.66 |

These results show that compared to the work function of 5.4eV exhibited by common hole transport materials such as NPD and TPD, the arylamine compounds represented by general formula (A) have adequate energy levels, good hole transport ability, and excellent electron blocking ability.

### [EXAMPLE 9]

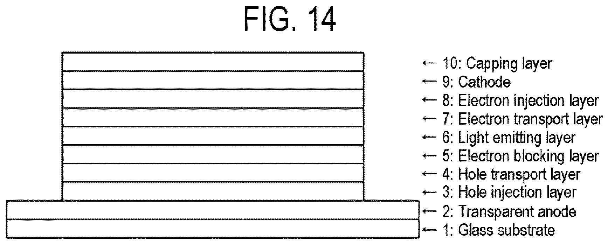

An organic EL element was fabricated by sequentially depositing, on a glass substrate 1 having a reflective ITO electrode serving as a transparent anode 2 formed thereon, a hole injection layer 3, a hole transport layer 4, an electron blocking layer 5, a light emitting layer 6, an electron transport layer 7, an electron injection layer 8, a cathode 9, and a capping layer 10 in this order, as shown in FIG. 14.

Specifically, as the transparent anode 2, an ITO film with a thickness of 50 nm, a reflective silver alloy film with a thickness of 100 nm, and an ITO film with a thickness of 5 nm were sequentially formed in this order on a glass substrate 1, which was then subjected to ultrasonic cleaning in isopropyl alcohol for 20 minutes followed by drying for 10 minutes on a hot plate heated to 250°C. Subsequently, after UV ozone treatment for 2 minutes, the glass substrate with ITO was installed in a vacuum deposition apparatus which was then evacuated to a pressure of 0.001 Pa or lower.

Next, as the hole injection layer 3, the electron acceptor (Acceptor-1) and the compound (HTM-1) of the following structural formulas were deposited by binary deposition at a deposition rate ratio of Acceptor-1:Compound (HTM-1) = 3:97 to form a film with a thickness of 10 nm, covering the transparent anode 2.

On the hole injection layer 3, a film of Compound (HTM-1) with a thickness of 140 nm was formed as the hole transport layer 4.

On the hole transport layer 4, Compound (3) from Example 1 was formed into a film with a thickness of 5 nm as the electron blocking layer 5.

On the electron blocking layer 5, the compound (EMD-1) and compound (EMH-1) of the following structural formulas were deposited by binary deposition at a deposition rate ratio of Compound (EMD-1):Compound (EMH-1) = 5:95 to form a film with a thickness of 20 nm as the light emitting layer 6.

On top of the light emitting layer 6, the compound (ETM-1) and compound (ETM-2) of the following structural formulas were deposited by binary deposition at a deposition rate ratio of Compound (ETM-1):Compound (ETM-2) = 50:50 to form a film with a thickness of 30 nm as the electron transport layer 7.

On the electron transport layer 7, lithium fluoride was deposited to a thickness of 1 nm to form the electron injection layer 8.

On the electron injection layer 8, a magnesium-silver alloy was deposited to a thickness of 12 nm to form the cathode 9.

Finally, as the capping layer 10, the compound (CPL-1) of the following structural formula was formed into a film with a thickness of 60 nm.

Characteristics of the thus fabricated organic EL element were measured under ambient atmosphere at an ordinary temperature.

Light emission characteristics measured upon application of a DC voltage to the fabricated organic EL element are summarized in Table 3.

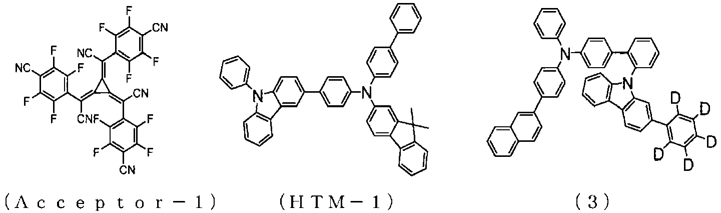

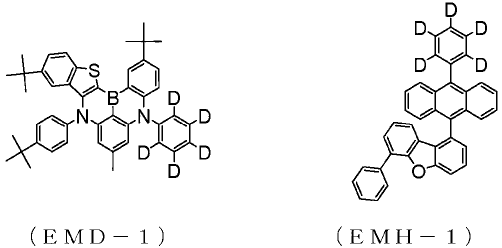

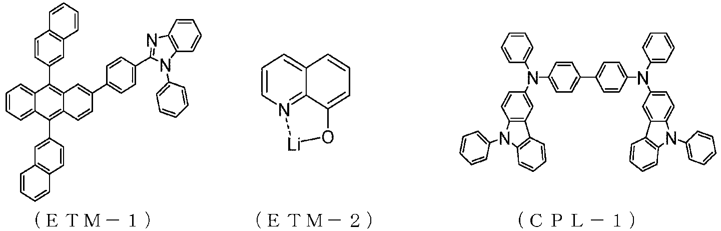

### [EXAMPLES 10 to 14]

Organic EL elements were fabricated in the same manner as in Example 9, with the exception of using the compounds obtained in Examples 2 to 6 individually, instead of Compound (3) of Example 1, as the material for the electron blocking layer 5. Characteristics of the thus fabricated organic EL elements were measured under ambient atmosphere at an ordinary temperature. Light emission characteristics measured upon application of a DC voltage to the fabricated organic EL elements are summarized in Table 3.

### [COMPARATIVE EXAMPLE 1]

For comparison, an organic EL element was fabricated in the same manner as in Example 9, with the exception of using the compound (HTM-2) of the following structural formula (*see* Patent Literature 7, for example), instead of Compound (3) of Example 1, as the material for the electron blocking layer 5. Characteristics of the thus fabricated organic EL element were measured under ambient atmosphere at an ordinary temperature. Light emission characteristics measured upon application of a DC voltage to the fabricated organic EL element are summarized in Table 3.

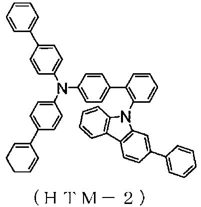

### [COMPARATIVE EXAMPLE 2]

For comparison, an organic EL element was fabricated in the same manner as in Example 9, with the exception of using the compound (HTM-3) of the following structural formula (*see* Patent Literature 8, for example), instead of Compound (3) of Example 1, as the material for the electron blocking layer 5. Characteristics of the thus fabricated organic EL element were measured under ambient atmosphere at an ordinary temperature. Light emission characteristics measured upon application of a DC voltage to the fabricated organic EL element are summarized in Table 3.

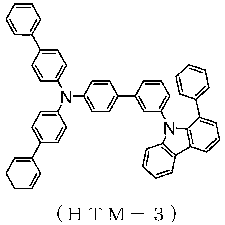

The lifetimes of the organic EL elements fabricated in Examples 9 to 14 and Comparative Examples 1 and 2 were measured. The results are summarized in Table 3. The lifetime was determined as the time taken for the luminance of 1000 cd/m² at the start of light emission (initial luminance) to decrease to 950 cd/m² (corresponding to 95% of the initial luminance, decay to 95%) under constant current operation.

**TABLE 3**

| | Electron blocking layer | Volt. [V] (@10mA/ cm2) | Luminance [cd/m2] (@10mA/cm2) | Luminous efficiency [cd/A] (@10mA/cm2) | Power efficiency [lm/W] (@10mA/cm2) | Device Lifetime (decay to 95%) |
|---|---|---|---|---|---|---|
| Ex. 9 | Cpd. (3) | 3.48 | 930 | 9.31 | 8.42 | 488 hours |
| Ex. 10 | Cpd. (24) | 3.47 | 887 | 8.87 | 8.02 | 463 hours |
| Ex. 11 | Cpd. (25) | 3.49 | 935 | 9.36 | 8.43 | 453 hours |
| Ex. 12 | Cpd. (26) | 3.46 | 950 | 9.52 | 8.66 | 450 hours |
| Ex. 13 | Cpd. (162) | 3.47 | 919 | 9.03 | 8.45 | 430 hours |
| Ex. 14 | Cpd. (163) | 3.32 | 841 | 8.61 | 8.27 | 425 hours |
| C. Ex. 1 | HTM-2 | 3.60 | 822 | 8.24 | 7.19 | 364 hours |
| C. Ex. 2 | HTM-3 | 3.57 | 826 | 8.26 | 7.28 | 349 hours |

As shown in Table 3, the luminous efficiency at a current density of 10 mA/cm² ranged from 8.61 to 9.52 cd/A for the organic EL elements in Examples 9 to 14, compared to 8.24 to 8.26 cd/A for the organic EL elements in Comparative Examples 1 and 2, indicating higher efficiencies. Similarly, the organic EL elements in Examples 9 to 14 had a power efficiency ranging from 8.02 to 8.66 lm/W, compared to 7.19 to 7.28 lm/W for those in Comparative Examples 1 and 2, again showing higher efficiencies. Furthermore, the device lifetime (decay to 95%) was extended significantly to 425 to 488 hours for the organic EL elements in Examples 9 to 14, compared to 349 to 364 hours for those in Comparative Examples 1 and 2.

As is clear from the results shown above, organic EL elements of the present disclosure, which use arylamine compounds having high hole mobility and excellent electron blocking ability, can achieve a higher luminous efficiency and an extended lifespan compared to conventional organic EL elements.

### [Description for Reference Numerals]

1: Glass substrate
2: Transparent anode
3: Hole injection layer
4: Hole transport layer
5: Electron blocking layer
6: Light emitting layer
7: Electron transport layer
8: Electron injection layer
9: Cathode
10: Capping layer

### INDUSTRIAL APPLICABILITY

Organic EL elements of the present disclosure employing an aryl amine compound having a specific structure have improved durability along with enhanced luminous efficiency, enabling their use in applications such as home electronics and lighting equipment.

## Claims

1. An arylamine compound represented by general formula (A): 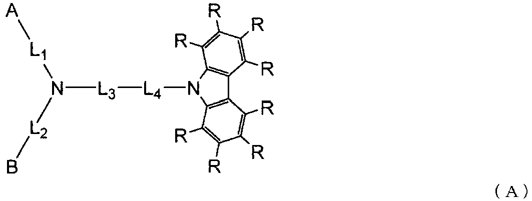 wherein:
A and B each represents a substituted or unsubstituted aromatic hydrocarbon group, a substituted or unsubstituted aromatic heterocyclic group, or a substituted or unsubstituted fused polycyclic aromatic group;
L₁ to L₄ each represents a single bond, a substituted or unsubstituted divalent aromatic hydrocarbon group, a substituted or unsubstituted divalent aromatic heterocyclic group, or a substituted or unsubstituted divalent fused polycyclic aromatic group; and
Rs may be the same or different, and each of the Rs represents a hydrogen atom, a deuterium atom, a substituted or unsubstituted aromatic hydrocarbon group, a substituted or unsubstituted aromatic heterocyclic group, or a substituted or unsubstituted fused polycyclic aromatic group, provided that at least one of the Rs is a substituted aromatic substituent.

2. The arylamine compound of claim 1, wherein at least one of the Rs in general formula (A) represents a deuterated phenyl group.

3. The arylamine compound of claim 2, wherein the compound is represented by general formula (B), (C), (D), or (E): 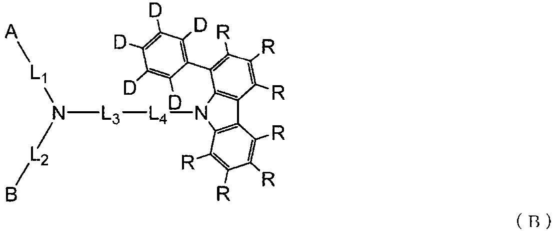 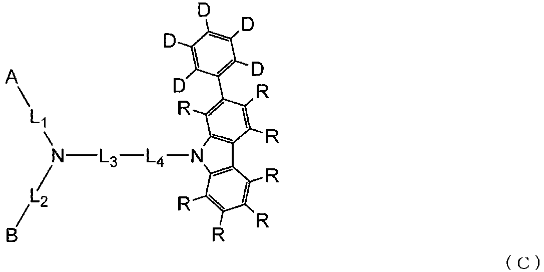 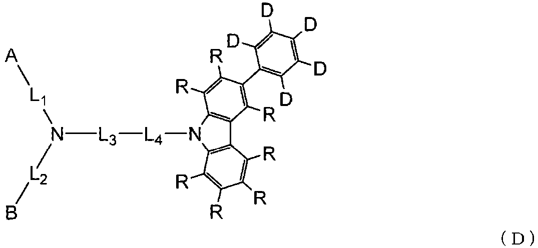 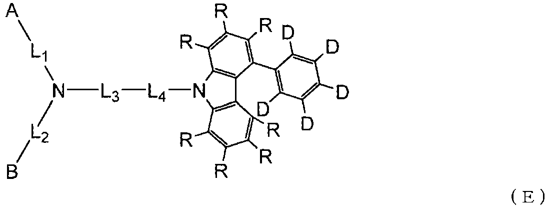 wherein
A, B, R, and L₁ to L₄ in general formula (B), (C), (D), or (E) are as defined in general formula (A).

4. The arylamine compound of claim 3, wherein R in general formula (B), (C), (D), or (E) represents a hydrogen atom or a deuterium atom.

5. The arylamine compound of claim 4, wherein L₁ to L₄ in general formula (B), (C), (D), or (E) each represents a single bond, a substituted or unsubstituted phenylene group, a substituted or unsubstituted naphthylene group, or a substituted or unsubstituted biphenylene group.

6. The arylamine compound of claim 5, wherein L₄ in general formula (B), (C), (D), or (E) represents a substituted or unsubstituted 1,2-phenylene group, a substituted or unsubstituted 1,3-phenylene group, a substituted or unsubstituted 1,4-phenylene group, a substituted or unsubstituted 1,2-naphthylene group, a substituted or unsubstituted 1,3-naphthylene group, or a substituted or unsubstituted 1,4-naphthylene group.

7. The arylamine compound of claim 6, wherein L₄ in general formula (B), (C), (D), or (E) represents an unsubstituted 1,2-phenylene group, an unsubstituted 1,3-phenylene group, an unsubstituted 1,4-phenylene group, an unsubstituted 1,2-naphthylene group, an unsubstituted 1,3-naphthylene group, or an unsubstituted 1,4-naphthylene group.

8. An organic EL element having a pair of electrodes and at least one organic layer sandwiched therebetween, wherein the organic layer comprises the arylamine compound of claim 1 or 2.

9. The organic EL element of claim 8, wherein the organic layer is a hole transport layer.

10. The organic EL element of claim 8, wherein the organic layer is an electron blocking layer.

11. The organic EL element of claim 8, wherein the organic layer is a hole injection layer.

12. The organic EL element of claim 8, wherein the organic layer is a light emitting layer.

13. An electronic device having a pair of electrodes and at least one organic layer sandwiched therebetween, wherein the organic layer comprises the arylamine compound of claim 1 or 2.
